Europäisches Patentamt

European Patent Office

Office européen des brevets

(19)

(11) Veröffentlichungsnummer: **0 030 735**
**B1**

(12) **EUROPÄISCHE PATENTSCHRIFT**

(45) Veröffentlichungstag der Patentschrift:
19.06.85

(51) Int. Cl.⁴: **G 01 N 33/53**

(21) Anmeldenummer: 80107928.6

(22) Anmeldetag: 16.12.80

(54) **Reagenzien für Radioimmunverfahren.**

(30) Priorität: 17.12.79 SE 7910382

(43) Veröffentlichungstag der Anmeldung:
24.06.81 Patentblatt 81/25

(45) Bekanntmachung des Hinweises auf die Patenterteilung:
19.06.85 Patentblatt 85/25

(84) Benannte Vertragsstaaten:
BE CH DE GB LI NL SE

(56) Entgegenhaltungen:
DE - A - 2 261 561
DE - A - 2 736 910
US - A - 4 001 583

ZEITSCHRIFT FÜR ANGEWANDTE CHEMIE, 88.
Jahrgang, Nr. 17, 1976 H.G. ECKERT "Die Technik des
Radioimmunoassays" Seiten 565 bis 573

(73) Patentinhaber: Thorell, Jan I., Beleshogsvagen 1,
S-21618 Malmö (SE)

(72) Erfinder: Thorell, Jan I., Beleshogsvagen 1,
S-21618 Malmö (SE)

(74) Vertreter: Lederer, Franz, Dr. et al, Patentanwälte Dr.
Franz Lederer Reiner F. Meyer Lucile-Grahn-Strasse 22,
D-8000 München 80 (DE)

## Beschreibung

Die vorliegende Erfindung betrifft neue Abschirmmittel, welche als Reagenzien für Radioimmunverfahren, bei denen die Abschirmung in der Probe selbst erfolgt, verwendet werden.

Radioimmunverfahren und andere Radioligandverfahren erfordern einen abschließenden Trennungsschritt, bei dem freier und gebundener Radioligand in verschiedene Phasen getrennt werden, um das Messen der Radioaktivität in einer dieser Phasen zu ermöglichen. In den meisten Verfahren bedingt diese Trennung zwei Schritte, nämlich erstens eine Zentrifugation und dann zweitens die Entfernung der flüssigen Phase durch Dekantieren, Absaugen oder ähnliches. Diese Schritte sind arbeitsintensiv und stellen für die Entwicklung vollständig automatisierter Radioimmunverfahren Hindernisse dar.

Die Entfernung des Überstandes führt zu großen Schwierigkeiten hinsichtlich der Genauigkeit des Tests und hat das Risiko, daß die Oberflächen der Teströhrchen verunreinigt werden. Ein anderer Weg, um diese Schwierigkeiten zu umgehen, besteht darin, gebundenen und freien Radioliganden in verschiedene Phasen zu trennen und dann die Strahlung einer dieser Phasen selektiv abzuschirmen, während beide Phasen sich im Teströhrchen befinden. Die energiemäßig kleine Gammastrahlung von $^{125}$J (27—35 keV), dem dominierenden Markierungsmittel bei Radioimmunverfahren, durchdringt Materialien mit hoher Dichte sehr spärlich. Durch Einschluß eines Abschirmmittels in das Verfahren wird es möglich, die Strahlung von einer der resultierenden Phasen aus dem Röhrchen zu verhindern.

Im US-Patent Nr. 4 158 135 wird eine Analysenmethode beschrieben, bei der die radioaktive Substanz zwischen einer flüssigen und einer festen Phase verteilt wird, wobei die Strahlung einer Phase gemessen wird, wogegen die Strahlung der anderen Phase abgeschirmt wird, indem dieser ein die Strahlung absorbierendes Mittel zugesetzt wird. In dieser Weise kann das Radioimmunverfahren ohne physikalische Trennung der beiden Phasen durchgeführt werden. Die Wahl eines geeigneten, die Strahlung absorbierenden Mittels hängt in starkem Maß von dem im Verfahren verwendeten radioaktiven Isotop ab. So kann zum Beispiel, wenn das weit verbreitete $^{125}$J verwendet wird, mit einer Vielzahl von Elementen, auch solchen mit einer niedrigen Ordnungszahl Z, eine effektive Abschirmung der Strahlung erreicht werden. Geeignete Elemente umfassen Silber, Cadmium, Wolfram und Wismut. Bei den im obenerwähnten US-Patent spezifisch beschriebenen Ausführungsformen wird Wolframpulver als Abschirmmittel verwendet.

Bei der Auswahl des geeigneten Abschirmmittels und dessen physikalischer Form ist es erwünscht, daß dieses Abschirmmittel mit der Antigen-Antikörperbindung nicht interferiert und daß das Abschirmmittel, sofern unlöslich, die Reaktanten des Verfahrens nicht auf seiner Oberfläche adsorbiert. Gemäß dem obenerwähnten US-Patent wird das Unlöslichmachen des gebundenen oder ungebundenen Radioliganden zum Zweck der Trennung dieser Komponenten mit an sich bekannten Techniken und Reagenzien durchgeführt, das heißt durch Doppelantikörpertrennung, chemische Ausfällung oder durch Verwendung von Antikörpern oder Antigenen die an partikelförmige, wasserunlösliche Träger gebunden sind. Es ist aber auch möglich, das radioaktive Material, das nicht reagiert hat, durch Adsorbtion an einen speziellen Adsorber unlöslich zu machen, wobei gewöhnlich Aktivkohle verwendet wird, welche vorzugsweise mit Dextran behandelt ist. Gemäß einem weiteren Aspekt des obenerwähnten US-Patentes kann die Aktivkohle mit dem Abschirmmittel vermischt werden. Schließlich kann gemäß dem obenerwähnten US-Patent ein Abschirmmittel verwendet werden, welches auch das radioaktive Material, welches nicht reagiert hat, adsorbiert. Ein Beispiel eines solchen Materials ist Wismutcarbonatpulver.

Die Aufgabe der vorliegenden Erfindung besteht darin, die erwähnten Radioimmunverfahren zu vereinfachen.

Die vorliegende Erfindung betrifft neue Abschirmmittel, wie sie in Anspruch 1 beansprucht sind.

Bevorzugte Ausgestaltungen sind in den abhängigen Ansprüchen zu finden.

Gemäß einem Aspekt der vorliegenden Erfindung ist die zweite aktive Komponente aus Materialien ausgewählt, welche entweder mit dem Reagens oder dem Reaktionsprodukt des Immunverfahrens immunologisch reagieren. Geeignete Materialien umfassen Antikörper (erster Antikörper), welche für die im Radioimmunverfahren zu bestimmenden Substanzen spezifisch sind, Antigene und Antikörper (zweiter Antikörper), welche in einem zweiten Wirtstier erzeugt wurden und gegen die Seren des Wirtstieres gerichtet sind, in welchem der erste Antikörper gebildet wurde. Die zweite aktive Komponente kann auch ein biologisch aktives Bindemittel vom Nicht-Immuntyp sein, wie z. B. Transportproteine, z. B. thyroxinbindendes Globulin, oder Rezeptoren cellulären Ursprungs, wie cytoplasmatische Östrogenrezeptoren oder Hormonrezeptoren aus der Plasmamembran.

Die erste aktive Komponente kann ein der Fachwelt bekanntes Abschirmmittel sein, welches eine hohe Fähigkeit aufweist, die durch einen Radioliganden hervorgerufene Strahlung zu absorbieren. Im speziellen umfassen diese Materialien die bereits früher für diese Zwecke beschriebenen Abschirmmittel, das heißt Silber, Cadmium, Wolfram, Wismut oder Verbindungen, welche diese Elemente enthalten, wie die Oxide davon. Eine besonders bevorzugte erste aktive Komponente enthält Wismut, besonders bevorzugt Wismutoxid ($Bi_2O_3$).

Die Trägermatrix umfaßt solche Matrices, welche nach herkömmlicher Weise zur Immobilisierung biologisch aktiver Moleküle verwendet werden, und können aus Gelen, Glas oder polymerem Material erhalten werden. Bevorzugte Matrixmaterialien für diesen Zweck umfassen Agarose, Polyacrylamid, Stärke oder Polyvinylidenfluorid.

Ein geeignetes Verfahren zur Herstellung einer Komponente gemäß vorliegender Erfindung besteht darin, das Matrixmaterial in Gegenwart der ersten aktiven Komponente zu insolubilisieren, gelieren oder polymerisieren, um Partikel, vorzugsweise Mikropartikel, des Polymermaterials zu erhalten, welche die erste aktive Komponente enthalten. Die zweite aktive Komponente wird darauf kovalent an die Oberfläche der Partikel gebunden oder kovalent an die Matrix gebunden oder wird durch die Matrix eingefangen, wobei bekannte Arten zum Kuppeln oder Einfangen oder anderweitigem Immobilisieren biologisch aktiver Substanzen an feste Träger verwendet werden.

Die Größe und die Eigenschaften der Partikel sind zu einem großen Teil von den Eigenschaften der ersten aktiven Komponente abhängig. Es ist bevorzugt, daß diese Komponente in mikrokristalliner Form ist, wobei die größten Kristalle eine Dimension unterhalb 5 µm haben. Auch größere Partikel sind geeignet, doch liegt die am meisten bevorzugte Größe bei 2 µm Kristalldimension. So kann man zum Beispiel eine geeignete erste Komponente, wie Wismutoxid durch Polymerisieren des Acrylamidmonomers in Gegenwart von Wismutoxid in eine Polyacrylamidmatrix inkorporieren. Die Polymerisation wird zweckmäßig in einer wasser-organischen Lösungsmittel-Emulsion durchgeführt. Wegen der hohen Dichte der das Wismutoxid enthaltenden Partikel ist es wünschenswert, die Polymerisation unter Verwendung eines organischen Lösungsmittels mit hoher Dichte, wie Kohlenstofftetrachlorid, durchzuführen. Nach einem anderen Verfahrensaspekt wird ein organisches Lösungsmittel mit geringerer Dichte, wie Toluol, verwendet, wobei die Partikel während der Polymerisation sedimentiert werden.

Neben den obenerwähnten Komponenten enthält das Polymerisationsreaktionsgemisch ein herkömmliches, die Polymerisation des Monomers induzierendes Mittel, sowie ein herkömmliches Emulgiermittel, wie Manitanmonooleat, welches käuflich als Arlacel A von Serva, Heidelberg, erhältlich ist. Ein repräsentatives Polymerisationsgemisch enthält die folgenden Komponenten in Gewichtsteilen: 0,4 bis 2,0, vorzugsweise 1,9 Acrylamid; 0,2 bis 0,4, vorzugsweise 0,4 N,N'-Methylenbisacrylamid; 0,1 bis 2, vorzugsweise 1,0 Wismutoxid; ungefähr 10,0 Wasser; und eine organische Phase von 50 bis 250 ml Kohlenstofftetrachlorid, enthaltend 1 bis 4, vorzugsweise 2% Arlacel A. Die Polymerisation des Polyacrylamides zur Matrix wird durch 0,1 Teile einer 50%igen Ammoniumpersulphatlösung in Wasser und 0,4 bis 0,5 Teile N,N,N',N'-Tetrame- tyläthylendiamin aktiviert und bei einer Temperatur im Bereich von ungefähr 0 bis 10°C, vorzugsweise 2°C, durchgeführt.

Neben Polyacrylamid kommen als Matrix Agarose (1—5%, vorzugsweise 2%) oder Stärke (40—80%, vorzugsweise 60%) in Betracht. Die Agarose- oder Stärkematrix wird in heißer und geschmolzener Form mit der ersten aktiven Komponente gemischt, um eine homogene Suspension zu erhalten. Wie bei Polyacrylamid wird die vorerwähnte Suspension in einem wasser-organischen Lösungsmittelsystem in Partikel geliert. Tröpfchen des Stärke- oder Agarosegelproduktes oder des Polyacrylamidpolymers läßt man dann zu festen Partikeln der gewünschten Größe im Bereich von 1 bis 100 µm, vorzugsweise weniger als 10 µm, verfestigen. Diese Partikel haben eine Dichte im Bereich von 1,2 bis 8 g pro Kubikzentimeter. Um eine Trennung der Partikel während derer Verfestigung zu erreichen, wird die organische Phase des Lösungsmittelsystems geschüttelt, zum Beispiel durch Rühren oder durch Beschallen.

Eine andere geeignete Matrix ist Polyvinylidenfluorid (PVF). Ungefähr 0,4 bis 3 g, vorzugsweise 2 g PVF werden in 20 ml Dimethylformamid gelöst und mit 10 bis 50 g, vorzugsweise 40 g Wismutoxid gemischt. Tröpfchen des erhaltenen Materials läßt man zu kleinen Partikeln der gewünschten Größe im Bereich von 1 bis 100 µm verfestigen, was durch Zugabe dieses Materials in 200 bis 500 ml Isopropanol, Äthanol oder Wasser geschieht. Die Partikelgröße kann durch Homogenisation weiter reduziert werden.

Nach einem weiteren Verfahren zur Herstellung der Abschirmmittel gemäß der vorliegenden Erfindung wird die erste und die zweite aktive Komponente mit dem Gel oder dem Polymermaterial in geschmolzener, gelöster oder nicht polymerisierter Form gut durchmischt, worauf die Mischung dann ausgegossen oder anderweitig zu einer starren Matrix unlöslich gemacht wird, welche dann zum Beispiel durch Mahlen zu den Partikeln der gewünschten Größe fein verteilt wird. Es ist aber auch möglich, die Matrix erst grob zu zerteilen, dann in einer Wasserphase zu suspendieren und schließlich mit einem Homogenisator (zum Beispiel Polytron) zu den Partikeln der gewünschten Größe zu homogenisieren.

Unabhängig von der Methode zur Herstellung der Partikel kann es erwünscht sein, Partikel kleinerer Größe zu verwenden als die gemäß der jeweiligen Methode erhaltenen. In diesem Falle können die Partikel durch Sieben oder durch Sedimentation in einer Flüssigkeit oder in einem Luftstrom getrennt werden.

Um die gewünschte Sedimentationscharakteristik der jeweiligen Partikel zu erhalten, kann man der Suspension, in welcher die Partikel enthalten sind, oberflächenaktive Stoffe, wie Tween 20®, Dextranpolyäthylenglykol oder Proteine zusetzen, wobei die Konzentration dieser Stoffe zur Erzielung der Aggregation der Partikel in der Suspension dem Fachmann bekannt ist.

Das Kuppeln der zweiten aktiven Komponente zu den Partikeln, enthaltend die erste aktive Komponente (verteilt im Gel oder in der Polymermatrix), kann unter Verwendung eines Kupplungsmittels erfolgen. Als Kupplungsmittel eignet sich Bromcyan oder ein bivalentes Kupplungsmittel, wie ein Dialdehyd, vorzugsweise Glutaraldehyd, welcher als Bindeglied zwischen den reaktiven Gruppen an der Oberfläche der Matrix und den reaktiven Gruppen der zweiten aktiven Komponente agiert.

Wenn PVF als Matrix verwendet wird, kann die Kupplung der zweiten aktiven Komponente — in der Form eines Antiserums — durch einstündige Behandlung der mit Wismutoxid beschichteten PVF Partikel mit Isopropanol und anschließendes dreimaliges Waschen der Partikel mit 0,1 M Phosphatpuffer vom pH 7,0, enthaltend 0,15 Mol Natriumchlorid, erreicht werden. Darauf werden die Partikel während 24 Stunden bei Raumtemperatur inkubiert.

Nach einem Aspekt der vorliegenden Erfindung wird die Sedimentationsrate der Partikel, enthaltend die erste und die zweite aktive Komponente, wie sie durch herkömmliche Sedimentationsverfahren erhalten wird, speziell ausgewählt, um die Reaktionsrate der zweiten aktiven Komponente entsprechend anzupassen. Durch Auswahl einer Kombination von Partikelgröße und Partikeldichte innerhalb des obenerwähnten Bereiches kann die Sedimentationsrate der Partikel so spezifiziert werden, daß sie sich innerhalb einer bestimmten Zeitperiode von ungefähr 1 bis 180 Minuten, vorzugsweise 15 bis 30 Minuten, am Boden der Röhrchen absetzen. Die Bindungskapazität der zweiten Komponente der Partikel ist so groß, daß die erwünschte immunologische Reaktion während der Sedimentation der Partikel stattfindet.

Gemäß einer speziellen Ausführungsform der vorliegenden Erfindung werden die Partikel, die die erste und die zweite aktive Komponente enthalten, in getrocknete oder lyophilisierte Dosierungseinheiten gebracht. Jede Dosierungseinheit wird zu einer Tablette verpreßt. Die Tabletten enthalten auch Füllstoffe, die notwendig sind, um die Tablettenform aufrechtzuerhalten und ihre Desintegration beim Suspendieren in Wasser zu erhöhen. Derartige Tablettenzusammensetzungen sind in der pharmazeutischen Industrie bestens bekannt und enthalten Füllmaterialien, wie Stärke, Lactose oder Magnesiumcarbonat. Auf diese Weise wird die genaue Zugabe einer vorbestimmten Menge der aktiven Komponenten zu jedem Teströhrchen verbessert und vereinfacht.

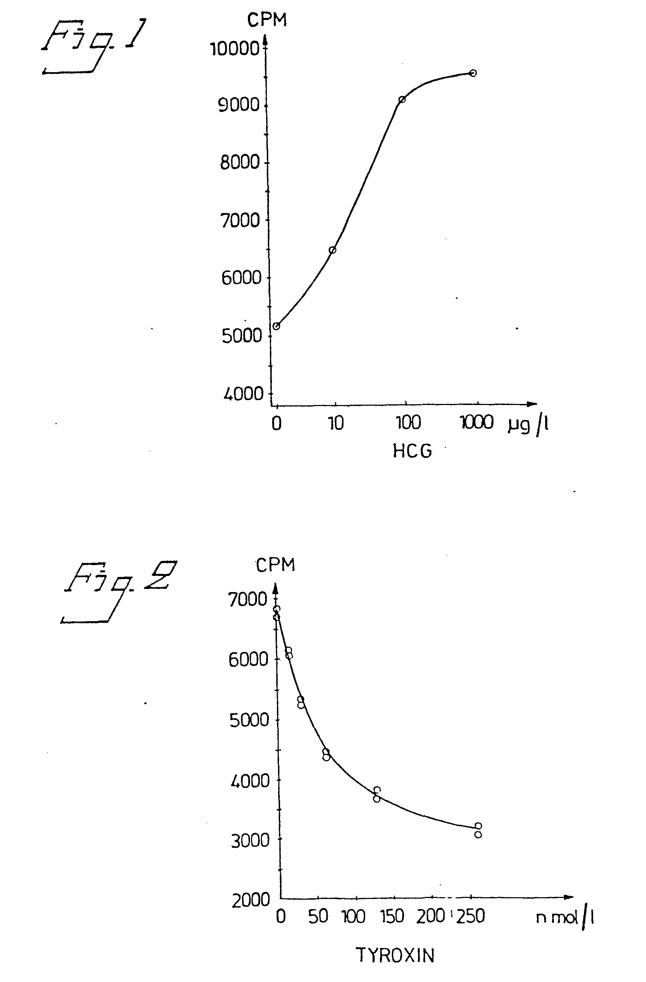

In einer spezifischen Ausführungsform dieses Aspektes der Erfindung wird ein Ziegenantikaninchenantikörper (zweiter Antikörper) unter Verwendung von Glutaraldehyd an Polyacrylamid-Partikel, in denen Wismutoxid dispergiert ist, gebunden. Das erhaltene Material wird in einem Radioimmunverfahren für menschliches Choriongonadotropin (HCG) verwendet, wobei eine Doppelantikörpermethode unter Festphasenabschirmung verwendet wird. Bei diesem Verfahren werden HCG-Standards (0,1 ml), $^{125}$J-HLH (0,2 ng) und Kaninchenantiserum gegen HCG (1 : 4000) in einem Gesamtvolumen von 0,5 ml während einer Stunde bei Raumtemperatur inkubiert. Dann wird 1 ml einer Suspension, enthaltend 0,1 ml Polyacrylamidpartikel mit zweitem Antikörper und Wismutoxid sowie 0,4 g Wismutoxid suspendiert in 1 ml Puffer zugegeben. Die Röhrchen werden während drei Stunden langsam rotiert. Daraufhin werden die Röhrchen während 15 Minuten zur Sedimentation stehengelassen und dann in einem Counter gezählt. Die erhaltenen Daten sind in der Standardkurve, die in Fig. 1 wiedergegeben wird, zusammengefaßt.

Nach einem anderen Verfahren werden Kaninchenantikörper gegen HCG an Wismutoxid enthaltende Polyacrylamidpartikel gebunden. In einem Inkubationsvolumen von 1 ml werden konstante Anteile solcher Partikel mit HCG Standards und $^{125}$J-HLH inkubiert. Die Röhrchen werden über Nacht langsam rotiert. Sie werden dann für 15 Minuten stehengelassen, damit die Partikel sedimentieren können. Sie werden dann in einem Counter gezählt. Die Ergebnisse sind im wesentlichen identisch mit denjenigen die in Fig. 1 dargestellt sind.

In einer weiteren Ausführungsweise wird als zweite aktive Komponente ein Material verwendet, welches mit der Radioligandkomponente des Reaktionsgemisches des Radioimmunverfahrens durch Adsorption reagiert. Bei dieser Ausführungsweise ist Aktivkohle die bevorzugte zweite aktive Komponente. Entsprechende Zusammensetzungen, enthaltend Aktivkohle und die erste aktive Komponente, sind durch Mischen dieser Komponenten mit einer Trägermatrix, wie Agarose, leicht erhältlich und können dann in die gewünschte Partikelgröße gebracht werden. Durch Herstellen und/oder Auswählen von Partikeln der gewünschten Größe und Dichte kann die Sedimentationsrate so gewählt werden, daß sich eine optimale Adsorption der Radioligandkomponente ergibt. In einer spezifischen Ausführungsweise wird Wismutoxid unter Verwendung von Agarose als Trägermatrix nach dem nachfolgenden Verfahren mit Aktivkohle überzogen. 100 g Wismutoxid werden mit 1 bis 100 g, vorzugsweise 2,5 g, Aktivkohle gemischt. 25 ml geschmolzene 1,5%ige Agarose wird zugegeben, und das Reaktionsgemisch wird bis zur Homogenität gemischt. Es wird über Nacht im Kühlschrank behalten. Das Gel wird in einem Mörser gründlich gemahlen. Das fragmentierte Gel wird in 250 ml 0,075 M Barbitalpuffer, enthaltend 0,25% Rinderserumalbumin und 0,05% Dextran T70 suspendiert. Selbstverständlich können neben Agarose auch die früher erwähnten Matrixmaterialien, wie Stärke, Polyacrylamid und PVF, mit Aktivkohle als zweiter aktiver Komponente verwendet werden. Die Aktivkohle wird mit dem Wismutoxid in den obenerwähnten Verhältnissen gemischt, wonach die Matrixpartikel so hergestellt werden, wie dies früher für Matrixpartikel beschrieben ist, die nur Wismutoxid enthalten.

1 ml dieser Suspension wird als Absorptions-Abschirmmittel in einem Radioimmunverfahren für Thyroxin (T₄) verwendet. Bei dieser Bestimmung werden 0,05 ml T₄ Standards mit 0,2 ml Kaninchenantiserum gegen T₄ (1 : 100) und 0,2 ml $^{125}$J-T₄ (200 pg) bei 4°C über Nacht inkubiert. Es wird eine Suspension von 0,4 g der Agarosepartikel, enthaltend die Aktivkohle und das Wismutoxid in 1 ml Puffer zugeführt. Die Röhrchen werden während 5 Sekunden geschüttelt und dann in aufrechter Position während 15 Minuten stehengelassen. Sie werden dann in einem automatischen Gamma-Zähler (LKB-Wallac Ultro Gamma II) gemessen. Die erhaltene Standardkurve ist aus Fig. 2 ersichtlich.

## Patentansprüche

1. Abschirmmittel, welche als Reagenzien für Radioimmunverfahren, bei denen die Abschirmung in der Probe selbst erfolgt, verwendet werden, dadurch gekennzeichnet, daß die einzelnen Partikel eine Größe von 1 bis 100 µm aufweisen und aus einer ersten und einer zweiten aktiven Komponente in Kombination mit einer Trägermatrix bestehen, wobei Partikelgröße und Partikeldichte so aufeinander abgestimmt sind, daß die Partikel eine Sedimentationsgeschwindigkeit von 1—180 Minuten aufweisen, und jedes dieser Partikel als erste aktive Komponente ein Abschirmmittel mit einer hohen Kapazität für die Absorption der von einem Radioliganden eines Radioimmunverfahrens ausgestrahlten Radioaktivität und als zweite aktive Komponente ein Material enthält, welches auf das Reagens oder das Reaktionsprodukt dieses Radioimmunverfahrens einwirkt.

2. Zusammensetzung nach Anspruch 1, dadurch gekennzeichnet, daß die zweite aktive Komponente ein Material ist, welches entweder mit dem Reagens oder dem Reaktionsprodukt des Radioimmunverfahrens immunologisch reagiert.

3. Zusammensetzung nach Anspruch 1 oder 2, dadurch gekennzeichnet, daß die erste aktive Komponente in einer Trägermatrix verteilt ist und daß die zweite aktive Komponente kovalent an die Oberfläche dieser Trägermatrix gebunden ist.

4. Zusammensetzung nach einem der Ansprüche 1, 2 oder 3, dadurch gekennzeichnet, daß die erste aktive Komponente Wismutoxid, die zweite aktive Komponente ein erster Antikörper und die Trägermatrix ein Polyacrylamid ist.

5. Zusammensetzung nach Anspruch 4, dadurch gekennzeichnet, daß die zweite aktive Komponente ein zweiter Antikörper ist.

6. Zusammensetzung nach Anspruch 2 oder 3, dadurch gekennzeichnet, daß die zweite aktive Komponente ein Antigen ist.

7. Zusammensetzung nach Anspruch 2 oder 3, dadurch gekennzeichnet, daß die zweite aktive Komponente ein Binder vom Nicht-Immuntyp aus der Gruppe der Transportproteine, thyroxinbindendes Globulin oder ein Rezeptor cellulären Ursprungs ist.

8. Zusammensetzung nach Anspruch 3, dadurch gekennzeichnet, daß die zweite aktive Komponente mittels Glutaraldehyd kovalent an die Trägermatrix gebunden ist.

9. Zusammensetzung nach Anspruch 1 oder 7, dadurch gekennzeichnet, daß die zweite aktive Komponente auf den Radioliganden des Radioimmunverfahrens durch Adsorption einwirkt.

10. Zusammensetzung nach Anspruch 1, dadurch gekennzeichnet, daß die erste aktive Komponente Wismutoxid, die zweite aktive Komponente Aktivkohle und die Trägermatrix Agarose, Stärke oder Polyvinylidenfluorid ist.

## Claims

1. Shielding agents intended to be used as reagents in radioimmuno-assays in which shielding takes place in the sample itself, characterized in that the individual particles present a size from 1 to 100 µm and consist of a first active component and a second active component in combination with a support matrix, the particle size and particle density being so adjusted to each other that the particles present a sedimentation rate amounting to 1—180 minutes, and each of these particles contains as first active component a shielding agent having a high capacity for absorption of the radiation emitted from a radioligand of a radioimmuno-assay and as second active component a material which acts on the reagent or the reaction product of said radioimmuno-assay.

2. A composition as claimed in claim 1, wherein the second active component represents a material capable of reacting immunologically with either the reagent or the reaction product of the radioimmuno-assay.

3. A composition as claimed in claim 1 or 2, wherein the first active component is distributed in a support matrix and the second active component is coupled by covalent bonds to the surface area of said support matrix.

4. A composition as claimed in claim 1, 2 or 3, wherein the first active component is bismuth oxide, the second active component is a first antibody and the support matrix is a polyacrylamide.

5. A composition as claimed in claim 4, wherein the second active component is a second antibody.

6. A composition as claimed in claim 2 or 3, wherein the second active component is an antigen.

7. A composition as claimed in claim 2 or 3, wherein the second active component is a non-immune type of binder from the group consisting of transport proteins, thyroxine-binding globulin and receptors of cellular origin.

8. A composition as claimed in claim 3, wherein the second active component is coupled by covalent bonds to the support matrix with glutaraldehyde.

9. A composition as claimed in claim 1 or 7, wherein the second active component reacts with the radioligand of the radioimmuno-assay by adsorption.

10. A composition as claimed in claim 1, wherein the first active component is bismuth oxide, the second active component is active carbon and the support matrix is agarose, starch or polyvinylidenefluoride.

## Revendications

1. Agents à effet d'écran, qui sont utilisés comme réactifs pour des procédés radio-immunologiques dans lesquels l'effet d'écran s'effectue dans l'échantillon lui-même, caractérisés en ce que les particules individuelles ont une grosseur de 1 à 100 μ et se composent d'un premier et d'un second constituants actifs en combinaison avec une matrice de support, les grosseurs de particules et la densité des particules sont accordées entre elles de telle manière que les particules présentent une vitesse de sédimentation de 1 à 180 minutes, et en ce que chacune de ces particules contient, en tant que premier constituant actif, un agent à effet d'écran ayant une capacité élevée d'absorption de la radioactivité émise par un radio-coordinat d'un procédé radio-immunologique et, en tant que second constituant actif, une matière qui agit sur le réactif ou sur le produit de réaction de ce procédé radio-immunologique.

2. Composition selon la revendication 1, caractérisée en ce que le second constituant actif est une matière qui réagit immunologiquement, soit avec le réactif, soit avec le produit de réaction du procédé radio-immunologique.

3. Composition selon la revendication 1 ou 2, caractérisée en ce que le premier constituant actif est réparti dans une matrice de support et en ce que le second constituant actif est lié par covalence à la surface de cette matrice de support.

4. Composition selon l'une quelconque des revendications 1 à 3, caractérisée en ce que le premier constituant actif est l'oxyde de bismuth, le second constituant actif est un premier anticorps et la matrice de support est un polyacrylamide.

5. Composition selon la revendication 4, caractérisée en ce que le second constituant actif est un second anticorps.

6. Composition selon la revendication 2 ou 3, caractérisée en le second constituant actif est un antigène.

7. Composition selon la revendication 2 ou 3, caractérisée en ce que le second constituant actif est un fixateur du type non-immun, choisi dans le groupe des protéines de transport, de la globuline fixant la thyroxine ou un récepteur d'origine cellulaire.

8. Composition selon la revendication 3, caractérisée en ce que le second constituant actif est lié par covalence à la matrice de support au moyen d'aldéhyde glutarique.

9. Composition selon la revendication 1 ou 7, caractérisée en ce que le second constituant actif agit par adsorption sur le radio-coordinat du procédé radio-immunologique.

10. Composition selon la revendication 1, caractérisée en ce que le premier constituant actif est l'oxyde de bismuth, le second constituant actif est un charbon actif et la matrice de support est l'agarose, l'amidon ou le fluorure de polyvinylidène.

*Fig. 1*

*Fig. 2*